Europäisches Patentamt

European Patent Office

Office européen des brevets

(11) Publication number: **0 427 877 B1**

(19)

# EUROPEAN PATENT SPECIFICATION

(12)

(45) Date of publication of patent specification: **16.06.93**　(51) Int. Cl.⁵: **A61K 9/70, A61L 15/16**

(21) Application number: **89120917.3**

(22) Date of filing: **10.11.89**

(54) **Adhesive tape preparation of clonidine.**

(43) Date of publication of application:
**22.05.91 Bulletin 91/21**

(45) Publication of the grant of the patent:
**16.06.93 Bulletin 93/24**

(84) Designated Contracting States:
**CH DE FR GB IT LI**

(56) References cited:
**EP-A- 0 295 777**
**US-A- 4 608 249**
**US-A- 4 765 974**
**US-A- 4 822 617**

**WPIL, FILE SUPPLIER, AN= 90-0040-27 (01),
Derwent Publications Ltd, London, GB; & JP-
A-01287024**

**PATENT ABSTRACTS OF JAPAN, vol. 12, no.
306 (C-522)[3153], 19th August 1988; & JP-
A-6379820**

(73) Proprietor: **NITTO DENKO CORPORATION
1-2, Shimohozumi 1-chome Ibaraki-shi
Osaka(JP)**

(72) Inventor: **Tokuda, Shoichi c/o Nitto Denki Cor-
poration
1-2 Shimohozumi 1-chome
Ibaraki-shi Osaka(JP)**
Inventor: **Otsuka, Saburo c/o Nitto Denki Cor-
poration
1-2 Shimohozumi 1-chome
Ibaraki-shi Osaka(JP)**
Inventor: **Ito, Yuusuke c/o Nitto Denki Cor-
poration
1-2 Shimohozumi 1-chome
Ibaraki-shi Osaka(JP)**

(74) Representative: **Patentanwälte Grünecker,
Kinkeldey, Stockmair & Partner
Maximilianstrasse 58
W-8000 München 22 (DE)**

**Description**

This invention relates to an adhesive tape preparation comprising a polytetrafluoroethylene porous sheet having moderate air permeability having thereon a layer containing clonidine as an active ingredient.

For the purpose of extending the duration of active ingredients and reducing side effects, a percutaneous administration route which makes it feasible to strictly control doses has recently been studied as a substitution for oral administration or injection. For example, percutaneous preparations including topical preparations of anti-inflammatory agents and systemic preparations of for example nitroglycerin, isosorbide dinitrate, scopolamine, estradiol have been developed.

Also with respect to clonidine acting as a hypotensive, a percutaneous preparation of clonidine which maintains a constant level in blood over 7 days or more thus achieving a prolonged duration has been proposed in place of oral tablets containing clonidine hydrochloride as described in MacGregor, T.R., et al., Clin. Pharmacol. Ther., Vol. 38(3), p. 278 (1985), U.S. Patent 4,202,211, and JP-A-54-20129 (the term "JP-A" as used herein means an "unexamined published Japanese patent application").

A dose of clonidine, given orally, usually lasts for about 8 h and therefore should be administered three times a day. This is a trouble to a patient, often causing the patient to forget to take the medicine, which may lead to serious symptoms due to break of the effect. For this reason, attention has been called to percutaneous preparations of clonidine. Conventional percutaneous preparations of clonidine comprise a support sheet substantially impermeable to clonidine having thereon a clonidine-containing layer, a layer controlling release of clonidine, and an adhesive layer for fixing to the skin in this order.

U.S. Patent 4,765,974 and JP-B-63-20806 (the term "JP-B" as used herein means an "examined published Japanese patent application") disclose percutaneous preparations comprising a support of various kinds having thereon an active ingredient-containing layer comprising a specific acrylate copolymer having dispersed therein clonidine and/or clonidine hydrochloride as an active ingredient with citric acid and/or succinic acid as a decomposition inhibitor.

JP-B-63-21647 discloses a percutaneous preparation of clonidine comprising a porous fluorine-containing resin film or sheet having a specific moisture permeability having thereon an active ingredient-containing layer.

While these conventional preparations for percutaneous administration of clonidine produce considerable effects in percutaneous absorption of clonidine, sustained clonidine release, and prevention of decomposition of clonidine during preservation (stability), no attention has been directed to suitability of the support. In particular, a support having no substantial air permeability would be of great irritation to the skin, giving rise to an extremely serious problem in practical application. JP-B-63-21647 supra suggests to use a porous fluorine-containing resin film having specific permeability to moisture as a support of low skin irritation, but sufficient study is not given to the optimum combination of components constituting the layer containing clonidine and also serving for fixing to the skin. In particular, since clonidine has a hypotensive effect it must be administered under strict dose control and, therefore, adhesion to the skin is one of important subjects to consider. That is, should the preparation be partly peeled apart from the skin due to poor adhesion, migration of clonidine through the skin is reduced, failing to obtain a sufficient hypotensive effect as expected.

Accordingly, in developing percutaneous preparations of an active ingredient requiring strict dose control (control on the amount percutaneously absorbed), such as clonidine, the following requirements should be filled. (1) The preparations must have sufficiently satisfactory adhesion to the skin. For example, peeling of even 10% of the area adhered is unaccepted. (2) The active ingredient should be stably present in the preparations, prevented from decomposition during long-term preservation. (3) In order to achieve sustained release of the active ingredient for a prolonged period of time, irritation to the skin should be minimized. For example, the skin irritation index (hereinafter defined) should not exceed 30. (4) Because clonidine is relatively expensive, it is desired to increase availability of the active ingredient. In greater detail, the rate of release to the skin surface should be at least 15%.

Under the present situation, percutaneous preparations of clonidine satisfying the above-described requirements have not yet been developed.

It is the object of the present application to provide an adhesive tape preparation of clonidine which achieves percutaneous administration of clonidine while satisfying various requirements stated above, such as adhesion to the skin and freedom from skin irritation.

As a result, it has now been found that an excellent clonidine preparation is obtained by forming a active ingredient-containing layer comprising an adhesive component having a specific composition and clonidine contained therein on a polytetrafluoroethylene porous sheet having specific air permeability.

The present invention provides an adhesive tape preparation of clonidine which comprises a polytetrafluoroethylene porous sheet having an air permeability, measured by means of a tester A type as specified in JIS P8117, of from 10 to 500 s having thereon an active ingredient-containing layer comprising a pressure-sensitive adhesive obtained by copolymerizing a monomer mixture comprising from 40 to 80% by weight of 2-ethylhexyl acrylate, from 20 to 60% by weight of 2-methoxyethyl acrylate, and from 0 to 40% by weight of vinyl acetate, the pressure-sensitive adhesive containing clonidine as an active ingredient.

When the monomer mixture comprises vinyl acetate, the active ingredient-containing layer preferably further contains succinic acid to prevent clonidine present in the adhesive tape preparation from decomposition during long-term preservation.

The polytetrafluoroethylene porous sheet which can be used for Supporting an active ingredient-containing layer in the present invention comprises polytetrafluoroethylene. In order to improve the mechanical strength, a sintered sheet is used. The porous sheet is generally prepared by uniaxially or biaxially stretching a polytetrafluoroethylene sheet simultaneously with sintering. A number of fine pores are formed by the stretching, and porosity, pore size and air permeability of the sheet can be controlled by stretch ratio adjustment.

The polytetrafluoroethylene porous sheet used in the present invention has an air permeability of from 10 to 500 s, preferably from 20 to 300 s. If the air permeability is less than 10 s, such high air permeability causes strike-through of clonidine during long-term preservation, decreasing percutaneous clonidine absorption below a practical level. If the air permeability exceeds 500 s, not only does skin irritativeness become strong, but the adhesiveness to the skin is reduced during long-term application.

The terminology "air permeability" as used herein means an average time (s) required for 100 ml of air to pass through the sheet having an area of 654 $mm^2$ as measured by means of a tester A type as specified in JIS P8117 "Testing Method of Air Permeability of Paper and Paperboard".

The porous sheet preferably has a thickness ranging from 5 to 300 $\mu$m, more preferably from 10 to 100 $\mu$m. A thin sheet is difficult to handle, and a thick sheet lacks in softness to cause a feeling of incompatibility during application to the skin.

The active ingredient-containing layer to be used in the present invention is required to stably support clonidine, the active ingredient, and to release the active ingredient through diffusive migration while exhibiting satisfactory adhesion to the skin for a long time without irritating the skin. It was found that a pressure-sensitive adhesive obtained by copolymerizing 2-ethylhexyl acrylate, 2-methoxyethyl acrylate, and vinyl acetate is suitable as an active ingredient-containing layer satisfying the above-described requirements. As a result of further investigation, it was ascertained that a copolymer obtained from a monomer mixture comprising from 40 to 80% by weight, preferably from 40 to 70% by weight, more preferably from 45 to 60% by weight, of 2-ethylhexyl acrylate; from 20 to 60% by weight, preferably from 20 to 50% by weight, more preferably from 25 to 40% by weight, of 2-methoxyethyl acrylate; and 0 to 40% by weight, preferably from 10 to 40% by weight, more preferably from 10 to 30% by weight, of vinyl acetate is more suitable. All the percent ratios of the monomers are based on the amount of the monomer mixture.

In particular, a copolymer obtained from a monomer mixture containing more than 40% by weight of vinyl acetate has turned out to reduce adhesion to the skin and also to give adverse influences to clonidine release properties.

If the ratio of 2-ethylhexyl acrylate is less than 40% by weight, the active ingredient-containing layer has poor clonidine release properties. If it exceeds 80% by weight, the cohesion of the layer is so reduced that the adhesive layer remains on the skin when stripped off the skin.

If the ratio of 2-methoxyethyl acrylate is less than 20% by weight, the layer has poor clonidine release properties, and if it exceeds 60% by weight, unfavorable gelation occurs during or after the copolymerization.

The pressure-sensitive adhesive used in the present invention can be prepared by any conventional methods such as radical polymerization. The pressure-sensitive adhesive preferably has a viscosity of from 5 to 25 Pa•s (50 to 250 poise) (25% by weight ethyl acetate solution at 30°C measured by a B type viscometer) and a weight average molecular weight of from 700,000 to 1,000,000 (determined by GPC).

Clonidine, an active ingredient having an action to reduce the tone of the central sympathetic nerve, is incorporated into the active ingredient-containing layer comprising the above-described pressure-sensitive adhesive preferably in an amount of from 1 to 25% by weight, more preferably from 2 to 10% by weight, based on the active ingredient-containing layer to thereby manifest hypotensive effects in the treatment or prevention of hypertension or migraine.

When the pressure-sensitive adhesive contains a vinyl acetate unit, it is preferred to incorporate succinic acid into the active ingredient-containing layer to prevent decomposition of clonidine because clonidine is particularly apt to be decomposed in the presence of vinyl acetate units. In this case, succinic acid is preferably added in an amount of from 0.1 to 5% by weight, more preferably from 0.3 to 4% by weight based on the active ingredient-containing layer. Addition of less than 0.1% by weight hardly produces significant effects. Addition of more than 4% by weight brings no further improvement on prevention of clonidine decomposition and is also unfavorable from the standpoint of skin irritation and cohesion of the layer.

For the purpose of increasing the amount of clonidine to be released, the active ingredient-containing layer can further contain one or more additives, such as glycols, e.g., propylene glycol, diethylene glycol, and triethylene glycol; N-methyl-2-pyrrolidone, N-methylpyrrolidone N-oxide, salicylic acid, urea, dimethyl sulfoxide, decylmethyl sulfoxide, diethyl sebacate, ethyl alcohol, and various surface active agents. From the viewpoint of adhesion to the skin and maintenance of a cohesive force of the layer, the amount of these additives to be used is preferably not more than 20% by weight, more preferably between 0.5 and 20% by weight, based on the total weight of the active ingredient-containing layer.

The thickness of the clonidine-containing layer is preferably adjusted within a range of from 5 to 300 $\mu$m, more preferably from 10 to 100 $\mu$m, taking adhesion to the skin, cohesion, releasability from the skin, and skin irritation into consideration.

In order to effectively relieve clonidine to the skin while minimizing skin irritation so as to have a skin irritation index of not higher than 30, it is desirable to adjust the moisture permeability of the whole preparation to a range of from 200 to 3,000 g/m$^2$•24 h. If the moisture permeability is below 200 g/m$^2$•24 h, the adhesive tape preparation applied to the skin is liable to cause stuffiness. As a result, the skin irritation becomes serious, and also the adhesion to the skin is likely to be reduced. On the other hand, if the moisture permeability exceeds 3,000 g/m$^2$•24 h, the effects of percutaneous administration by occlusive dressing technique (ODT) are reduced, sometimes failing to obtain sufficient pharmacological effects.

The terminology "moisture permeability" as used herein means a value obtained according to JIS Z0208 "Testing Methods for Determination of the Water Vapor Transmission Rate of Moisture-Proof Packaging Materials (Dish Method)". More specifically, the moisture permeability can be determined by placing a prescribed amount of anhydrous calcium chloride on the bottom of a moisture-permeable open top container, and the top is covered with the adhesive tape preparation of the present invention. The container is allowed to stand in a thermo-hygrostat set at a temperature of 40 ± 0.5°C and at a relative humidity of 90 ± 2% for 2 h, and an increase of weight of the container over that before standing in the thermo-hygrostat is measured to calculate the moisture permeability per 24 h.

The terminology "skin irritation index" as used herein means an index of primary skin irritation observed after the tape preparation of the present invention is applied to the skin of the human fore chest for 72 h as judged according to the Draize's standard (as described in J. Pharmacol. Exptl. Therap., vo..82, pp.377-390 (1944)). Specifically, a tape preparation having an area of 0.785 cm$^2$ (10 mm in diameter) is applied to the skin of the human chest of subjects for 72 h. The conditions of the skin, 30 min and 24 h after removing the preparation, are determined by the Draize's standard to keep the score. The above procedures are conducted twice, and using the larger score, the skin irritation index is determined by the following formula:

$$\text{skin irritation index} = \frac{\text{total score}}{\text{number of subjects}} \times 100$$

As described above, the adhesive tape preparation according to the present invention is a preparation for effectively administering clonidine through the skin. It achieves sustained release of clonidine and exhibits long-term preservability while minimizing skin irritation and maintaining sufficient adhesion to the skin. Hence, the adhesive tape preparation of the present invention produces excellent effects that have never been achieved by the conventional clonidine-containing percutaneous preparations.

The present invention is now illustrated in greater detail by way of the following Examples. Unless otherwise indicated, all percents are given by weight in these examples.

EXAMPLE 1

45 g of 2-ethylhexyl acrylate, 30 g of vinyl acetate, and 25 g of 2-methoxyethyl acrylate were charged in a flask in an inert gas atmosphere. To the mixture was added 0.1 g of benzoyl peroxide as a polymerization initiator. The reaction was continued for about 10 h under control by adjustment of the stirring speed and the external bath temperature and dropwise addition of ethyl acetate as a solvent to obtain a pressure-sensitive adhesive solution. The pressure-sensitive adhesive thus obtained had a viscosity of 13 to 16 Pa•s (130 to 160 poise) at 30°C as a 25% by weight solution and a weight average molecular weight of from 820,000 to 880,000.

Clonidine as an active ingredient and succinic acid were mixed with the resulting adhesive solution, and the composition was coated on a 60 $\mu$m thick polytetrafluoroethylene porous sheet having an air permeability of 20 s to a dry thickness of 50 $\mu$m to prepare an adhesive tape preparation according to the present invention. The active ingredient-containing layer had a clonidine concentration of 7.5% and a succinic acid concentration of 0.5%, and the adhesive tape preparation had a moisture permeability of 1,290 g/m$^2$•24 h.

EXAMPLE 2

60 g of 2-ethylhexyl acrylate, 20 g of vinyl acetate, and 20 g of 2-methoxyethyl acrylate were charged in a flask in an inert gas atmosphere, and 0.1 g of benzoyl peroxide as a polymerization initiator was added thereto to start polymerization. Following the same procedure as described in Example 1, a pressure-sensitive adhesive solution was obtained. The pressure-sensitive adhesive thus obtained had a viscosity of 9 to 11 Pa•s (90 to 110 poise) at 30°C as a 25% by weight solution and a weight average molecular weight of from 740,000 to 810,000.

Clonidine and succinic acid were mixed with the resulting adhesive solution, and the composition was coated on a 60 $\mu$m thick polytetrafluoroethylene porous sheet having an air permeability of 100 s to a dry thickness of 50 $\mu$m to prepare an adhesive tape preparation.

The active ingredient-containing layer of the resulting adhesive tape preparation had a clonidine concentration of 7.5% and a succinic acid concentration of 2%, and the adhesive tape preparation had a moisture permeability of 1,100 g/m$^2$•24 h.

EXAMPLE 3

Clonidine and succinic acid were mixed with the pressure-sensitive adhesive solution as obtained in Example 1, and the composition was coated on a 20 $\mu$m thick polytetrafluoroethylene porous sheet having an air permeability of 300 s to a dry thickness of 30 $\mu$m to prepare an adhesive tape preparation.

The active ingredient-containing layer of the adhesive tape preparation had a clonidine concentration of 10% and a succinic acid concentration of 1%, and the adhesive tape preparation had a moisture permeability of 1,540 g/m$^2$•24 h.

EXAMPLE 4

Clonidine and succinic acid were mixed with the pressure-sensitive adhesive solution as obtained in Example 2, and the composition was coated on a 100 $\mu$m thick polytetrafluoroethylene porous sheet having an air permeability of 200 s to a dry thickness of 100 $\mu$m to prepare an adhesive tape preparation.

The active ingredient-containing layer of the preparation had a clonidine concentration of 3% and a succinic acid concentration of 0.4%, and the preparation had a moisture permeability of 710 g/m$^2$•24 h.

EXAMPLE 5

75 g of 2-ethylhexyl acrylate and 25 g of 2-methoxyethyl acrylate were charged in a flask in an inert gas atmosphere, and 0.1 g of benzoyl peroxide was added thereto as a polymerization initiator to start polymerization. The same procedure as in Example 1 was followed to obtain a pressure-sensitive adhesive solution. The pressure-sensitive adhesive thus obtained had a viscosity of 8 to 10 Pa•s (80 to 100 poise) at 30°C as a 25% by weight solution and a weight average molecular weight of from 850,000 to 910,000.

Clonidine was mixed with the resulting adhesive solution, and the composition was coated on a 60 $\mu$m thick polytetrafluoroethylene porous sheet having an air permeability of 300 s to a dry thickness of 60 $\mu$m to obtain an adhesive tape preparation.

The active ingredient-containing layer of the resulting preparation had a clonidine concentration of 5%, and the preparation had a moisture permeability of 980 g/m$^2$•24 h.

EXAMPLE 6

55 g of 2-ethylhexyl acrylate, 15 g of vinyl acetate, and 30 g of 2-methoxyethyl acrylate were charged in a flask in a inert gas atmosphere, and 0.1 g of benzoyl peroxide as a polymerization initiator was added thereto to start polymerization. Following the same procedure as described in Example 1, a pressure-sensitive adhesive solution was obtained. The pressure-sensitive adhesive thus obtained had a viscosity of 15 to 17 Pa•s (150 to 170 poise) at 30°C as a 25% by weight solution and a weight average molecular weight of from 880,000 to 950,000.

Clonidine was mixed with the resulting adhesive solution, and the composition was coated on a 60 $\mu$m thick polytetrafluoroethylene porous sheet having an air permeability of 20 s to a dry thickness of 50 $\mu$m to prepare an adhesive tape preparation.

The active ingredient-containing layer of the resulting adhesive tape preparation had a clonidine concentration of 7.5%, and the adhesive tape preparation had a moisture permeability of 1,310 g/m$^2$•24 h.

COMPARATIVE EXAMPLE 1

An adhesive tape preparation was obtained in the same manner as in Example 1, except for replacing the polytetrafluoroethylene porous sheet with a 9 $\mu$m thick air-impermeable polyester film. The resulting adhesive tape preparation had a moisture permeability of 15 g/m$^2$•24 h.

COMPARATIVE EXAMPLE 2

An adhesive tape preparation was obtained in the same manner as in Example 2, except for replacing the polytetrafluoroethylene porous sheet with a 80 $\mu$m thick polyethylene porous sheet having an air permeability of 200 s. The resulting adhesive tape preparation had a moisture permeability of 1,000 g/m$^2$•24 h.

COMPARATIVE EXAMPLE 3

An adhesive tape preparation was obtained in the same manner as in Example 2, except for replacing the polytetrafluoroethylene porous sheet with a 60 $\mu$m thick polytetrafluoroethylene sheet which was substantially impermeable to air (air permeability: 36,000 s or more). The resulting adhesive tape preparation had a moisture permeability of 13 g/m$^2$•24 h.

COMPARATIVE EXAMPLE 4

An adhesive tape preparation was obtained in the same manner as in Example 1, except for replacing the polytetrafluoroethylene porous sheet with a 60 $\mu$m thick polytetrafluoroethylene sheet having an air permeability of 5 s. The resulting adhesive tape preparation had a moisture permeability of 2,380 g/m$^2$•24 h.

COMPARATIVE EXAMPLE 5

An adhesive tape preparation was obtained in the same manner as in Example 1, except for replacing the polytetrafluoroethylene porous sheet with a 60 $\mu$m thick polytetrafluoroethylene sheet having an air permeability of 700 s. The resulting adhesive tape preparation had a moisture permeability of 150 g/m$^2$•24 h.

Primary skin irritation, adhesion to the skin, migration of clonidine to the skin, and clonidine preservation stability of each of the adhesive tape preparations obtained in Examples 1 to 6 and Comparative Examples 1 to 5 were evaluated according to the following test methods, and the results obtained are shown in Table 1 below.

1) Primary Skin Irritation:

A tape preparation having an area of 0.785 cm$^2$ (10 mm in diameter) was applied to the skin of the human chest of subjects for 72 h. The conditions of the skin, 30 min and 24 h after removing the preparation, were determined by the Draize's standard to keep the score. The above procedures were conducted twice, and using the larger score, the sking irritation index was determined by the following formula:

6

$$\text{skin irritation index} = \frac{\text{total score}}{\text{number of subjects}} \times 100$$

2) Adhesion to the Skin:

The preparation sample was applied to the skin of the human fore chest for 72 h, and the rate of adhesion (%) was calculated from the area adhered immediately after the application and that after the 72 h application.

3) Rate of Migration to the Skin:

The preparation sample was applied to the shaved back of a rabbit for 24 h. The concentration of residual clonidine in the preparation was measured to obtain a rate of migration to the skin (%).

4) Preservation Stability:

The preparation sample was preserved at 40°C for 1 year, and the concentration of residual clonidine in the preparation was measured to obtain a clonidine retention (%).

## TABLE 1

| Example No. | Skin irritation index | Rate of adhesion (%) | Rate of clonidine migration (%) | Rate of clonidine retention (%) |
|---|---|---|---|---|
| Example 1 | 5 | 99.4 | 21 | 99.8 |
| " 2 | 9 | 99.2 | 27 | 99.7 |
| " 3 | 12 | 98.5 | 33 | 99.9 |
| " 4 | 15 | 99.0 | 28 | 99.7 |
| " 5 | 12 | 98.7 | 27 | 98.7 |
| " 6 | 8 | 99.0 | 31 | 96.5 |
| Comparative Example 1 | 42 | 54.2 | 57 | 99.8 |
| " 2 | 10 | 99.4 | 28 | 90.3 |
| " 3 | 48 | 53.7 | 52 | 99.5 |
| " 4 | 6 | 99.4 | 12 | 93.4 |
| " 5 | 32 | 90.0 | 40 | 99.5 |

It is understood from the results in Table 1 above that the adhesive tape preparations according to the present invention are excellent in adhesion property to the skin, stability of the active ingredient in the preparations, sustained release of the active ingredient, and availability of the active ingredient.

**Claims**

1. An adhesive tape preparation of clonidine which comprises a polytetrafluoroethylene porous sheet having an air permability measured by means of a tester A type as specified in JIS P8117, of from 10 to 550 s having thereon an active ingredient-containing layer comprising a pressure-sensitive adhesive obtained by copolymerising a monomer mixture comprising from 40 to 80% by weight of 2-ethylhexyl acrylate, from 20 to 60% by weight of 2-methoxyethyl acrylate, and 0 to 40% by weight of vinyl acetate, said pressure-sensitive adhesive containing clonidine as an active ingredient.

2. The adhesive tape preparation of claim 1, wherein said preparation has a moisture permeability, measured as specified in JIS Z0208, of from 200 to 3,000 g/m$^2$; 24 h.

3. The adhesive tape preparation of claim 1, wherein the polytetrafluoroethylene porous sheet has a thickness of from 5 to 300 $\mu$m.

4. The adhesive tape preparation of claim 1, wherein said polytetrafluoroethylene porous sheet has an air permeability of from 20 to 300 s.

5. The adhesive tape preparation of claim 1, wherein clonidine is present in a concentration of from 1 to 25% by weight based on the active ingredient-containing layer.

6. The adhesive tape preparation of claim 1, wherein said active ingredient-containing layer has a thickness of from 5 to 300 $\mu$m.

7. The adhesive tape preparation of claim 1, wherein said active ingredient-containing layer further contains succinic acid.

8. The adhesive tape preparation of claim 7, wherein said succinic acid is present in a concentration of from 0.1 to 5% by weight based on the active ingredient-containing layer.

9. The adhesive tape preparation of any of claims 1 to 8, wherein said active ingredient-containing layer comprises a pressure-sensitive adhesive obtained by copolymerizing a monomer mixture comprising from 40 to 70% by weight of 2-ethylhexyl acrylate, from 20 to 50% by weight of 2-methoxyethyl acrylate, and 10 to 40% by weight of vinyl acetate.

**Patentansprüche**

1. Klebeband- bzw. Klebstreifenzubereitung aus Clonidin, umfassend eine poröse Bahn bzw. Schicht aus Polytetrafluorethylen mit einer Luftdurchlässigkeit, gemessen mittels einer Testvorrichtung vom A-Typ, wie in JIS P8117 angegeben, von 10 bis 550 s mit einer darauf befindlichen Schicht, die einen Wirkstoff enthält, welche einen druckempfindlichen Klebstoff, erhalten durch Copolymerisieren einer Monomermischung, umfassend 40 bis 80 Gew.% 2-Ethylhexylacrylat, 20 bis 60 Gew.% 2-Methoxyethylacrylat und 0 bis 40 Gew.% Vinylacetat, umfaßt, wobei der druckempfindliche Klebstoff Clonidin als Wirkstoff enthält.

2. Klebebandzubereitung nach Anspruch 1, worin die Zubereitung eine Feuchtigkeitsdurchlässigkeit, gemessen, wie in JIS ZO208 angegeben, von 200 bis 3000 g/m$^2$ x 24 h besitzt.

3. Klebebandzubereitung nach Anspruch 1, worin die poröse Bahn bzw. Schicht aus Polytetrafluorethylen eine Dicke von 5 bis 300 $\mu$m besitzt.

4. Klebebandzubereitung nach Anspruch 1, worin die poröse Bahn bzw. Schicht aus Polytetrafluorethylen eine Luftdurchlässigkeit von 20 bis 300 s besitzt.

5. Klebebandzubereitung nach Anspruch 1, worin Clonidin in einer Konzentration von 1 bis 25 Gew.%, bezogen auf die Schicht, die den Wirkstoff enthält, vorliegt.

**6.** Klebebandzubereitung nach Anspruch 1, worin die Schicht, die den Wirkstoff enthält, eine Dicke von 5 bis 300 $\mu$m besitzt.

**7.** Klebebandzubereitung nach Anspruch 1, worin die Schicht, die den Wirkstoff enthält, weiterhin Bernsteinsäure enthält.

**8.** Klebebandzubereitung nach Anspruch 7, worin die Bernsteinsäure in einer Konzentration von 0,1 bis 5 Gew.%, bezogen auf die Schicht, die den Wirkstoff enthält, vorliegt.

**9.** Klebebandzubereitung nach einem der Ansprüche 1 bis 8, worin die Schicht, die den Wirkstoff enthält, einen druckempfindlichen Klebstoff, erhalten durch Copolymerisieren einer Monomermischung, umfassend 40 bis 70 Gew.% 2-Ethylhexylacrylat, 20 bis 50 Gew.% 2-Methoxyethylacrylat und 10 bis 40 Gew.% Vinylacetat, umfaßt.

**Revendications**

**1.** Préparation de clonidine sur ruban adhésif qui comprend une feuille poreuse de polytétrafluoroéthylène ayant une perméabilité à l'air de 10 à 550 s mesurée au moyen d'un appareil d'essai A spécifié dans la nonne japonaise JIS P8117, portant une couche contenant l'ingrédient actif comprenant un adhésif sensible à la pression obtenu par copolymérisation d'un mélange de monomères comprenant de 40 à 80% en poids d'acrylate de 2-éthylhexyle, de 20 à 60% en poids d'acrylate de 2-méthoxyéthyle et de 0 à 40% en poids d'acétate de vinyle, ledit adhésif sensible à la pression contenant de la clonidine comme ingrédient actif.

**2.** Préparation sur ruban adhésif selon la revendication 1, selon laquelle ladite préparation a une perméabilité à l'humidité de 200 à 3 000 g/m$^2$.24 h, mesurée selon les spécifications de la norme japonaise JIS Z0208.

**3.** Préparation sur ruban adhésif selon la revendication 1, dans laquelle la feuille poreuse de polytétrafluoroéthylène a une épaisseur de 5 à 300 $\mu$m.

**4.** Préparation sur ruban adhésif selon la revendication 1, dans laquelle ladite feuille poreuse de polytétrafluoroéthylène a une perméabilité à l'air de 20 à 300 s.

**5.** Préparation sur ruban adhésif selon la revendication 1, dans laquelle la clonidine est présente à une concentration de 1 à 25% en poids par rapport à la couche contenant l'ingrédient actif.

**6.** Préparation sur ruban adhésif selon la revendication 1, dans laquelle ladite couche contenant l'ingrédient actif a une épaisseur de 5 à 300 $\mu$m.

**7.** Préparation sur ruban adhésif selon la revendication 1, dans laquelle ladite couche contenant l'ingrédient actif contient en outre de l'acide succinique.

**8.** Préparation sur ruban adhésif selon la revendication 7, dans laquelle l'acide succinique est présent à une concentration de 0,1 à 5% en poids par rapport à la couche contenant l'ingrédient actif.

**9.** Préparation sur ruban adhésif selon l'une quelconque des revendications 1 à 8, dans laquelle ladite couche contenant l'ingrédient actif comprend un adhésif sensible à la pression obtenu en copolymérisant un mélange de monomères comprenant de 40 à 70% en poids d'acrylate de 2-éthylhexyle de 20 à 50% en poids d'acrylate de 2-méthoxyéthlyle et de 10 à 40% en poids d'acétate de vinyle.